(19) **Europäisches Patentamt European Patent Office Office européen des brevets**

(11) **EP 4 714 354 A1**

(12) **EUROPEAN PATENT APPLICATION**

(43) Date of publication:
25.03.2026 Bulletin 2026/13

(21) Application number: 25203761.9

(22) Date of filing: 22.09.2025

(51) International Patent Classification (IPC):
*A61B 5/287* (2021.01)  *A61B 5/367* (2021.01)
*A61B 5/00* (2006.01)  *A61B 18/14* (2006.01)

(52) Cooperative Patent Classification (CPC):
**A61B 5/287; A61B 5/367; A61B 5/6859;
A61B 18/1492**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **23.09.2024 US 202463697933 P
19.09.2025 US 202519334607**

(71) Applicant: **Biosense Webster (Israel) Ltd.
2066717 Yokneam (IL)**

(72) Inventors:
• **TSOREF, Liat
2066717 Yokneam (IL)**
• **ROGRIGUEZ, Haim
2066717 Yokneam (IL)**
• **PFEFFER, Shmuel Yitzhak
2066717 Yokneam (IL)**

(74) Representative: **Carpmaels & Ransford LLP
One Southampton Row
London WC1B 5HA (GB)**

(54) **IMPROVED CHARACTERIZATION OF SCAR TISSUE AT VARIED DEPTHS BASED ON SIGNALS DERIVED FROM DIFFERENT ELECTRODES**

(57)    Electrical activity is sensed from a plurality of electrodes of a multi-electrode catheter. For each of a plurality of surface locations of cardiac tissue, a processor identifies first electrical signals originating at a first depth and second electrical signals originating at a second depth, estimates a first near-field signal originating at the first depth for each of the first electrical signals and detects scar borders at the first depth based on the first near-field signals, and estimates a second near-field signal originating at the second depth for each of the second electrical signals and detects scar borders at the second depth based on the second near-field signals. Based on the scar borders detected at the first and second depths, the processor visually displays first and second maps of the heart depicting scar tissue at the first and second depths, respectively.

EP 4 714 354 A1

**Description**

Related Applications

The present disclosure is directed to several improved techniques for analyzing cardiac signals, and claims priority to U.S. Provisional Patent Application No. 63/697,933, filed September 23, 2024, entitled "Methods For Analyzing Cardiac Signals And Generating Electro-Anatomical Maps Of The Heart," incorporated herein by reference in its entirety. The disclosed techniques also relate to build on concepts discussed in pending U.S. Patent Application No. 18/072,793, filed December 1, 2022, entitled "Intracardiac Unipolar Far Field Cancelation Using Multiple Electrode Catheters" (pending), U.S. Patent Application No. 18/756,903, filed June 27, 2024, entitled "Intracardiac Unipolar Far Field Cancelation Using Multiple Electrode Catheters And Methods For Creating An Ecg Depth And Radial Lens" (pending), U.S. Patent Application No. 19/238,791, filed June 16, 2025, entitled "System and Method for Far-field Voltage Mapping for Scar Severity Estimation" and U.S. Patent Application No. 18/505,956 entitled "Catheter With Flexible Polymer As Outer Support Structure," filed November 9, 2023 (pending). These existing applications are all incorporated in their entirety by reference, and provide relevant background to the techniques disclosed herein. The improvements disclosed herein may be used in conjunction with techniques disclosed in the above applications.

Technical Field

This disclosure is related to generating, visualizing, and displaying information representative of cardiac signals.

Background

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., pulsed field or radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The electrodes may be placed in contact with cardiac tissue or other vascular tissue and then activated with RF, pulsed field or other energy to thereby ablate the contacted tissue. In some cases, the electrodes may be bipolar. In some other cases, a monopolar electrode may be used in conjunction with a ground pad or other reference electrode that is in contact with the patient.

Examples of ablation catheters are described in U.S. Pub. No. 2013/0030426, entitled "Integrated Ablation System using Catheter with Multiple Irrigation Lumens," published Jan. 31, 2013, the disclosure of which is incorporated by reference herein in its entirety; U.S. Pub. No. 2017/0312022, entitled "Irrigated Balloon Catheter with Flexible Circuit Electrode Assembly," published Nov. 2, 2017, the disclosure of which is incorporated by reference herein in its entirety; U.S. Pub. No. 2018/0071017, entitled "Ablation Catheter with a Flexible Printed Circuit Board," published Mar. 15, 2018, the disclosure of which is incorporated by reference herein in its entirety; U.S. Pub. No. 2018/0056038, entitled "Catheter with Bipole Electrode Spacer and Related Methods," published Mar. 1, 2018, the disclosure of which is incorporated by reference herein in its entirety; U.S. Pat. No. 10,130,422, entitled "Catheter with Soft Distal Tip for Mapping and Ablating Tubular Region," issued Nov. 20, 2018, the disclosure of which is incorporated by reference herein in its entirety; U.S. Pat. No. 8,956,353, entitled "Electrode Irrigation Using Micro-Jets," issued Feb. 17, 2015, the disclosure of which is incorporated by reference herein in its entirety; and U.S. Pat. No. 9,801,585, entitled "Electrocardiogram Noise Reduction," issued Oct. 31, 2017, the disclosure of which is incorporated by reference herein in its entirety.

Some catheter ablation procedures may be performed after using electrophysiology (EP) mapping to identify tissue regions that should be targeted for ablation. Such EP mapping may include the use of sensing microelectrodes on a catheter (e.g., the same catheter that is used to perform the ablation or a dedicated mapping catheter). Such sensing microelectrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued Apr. 14, 1998, the disclosure of which is incorporated by reference herein in its entirety. Examples of EP mapping catheters are described in U.S. Pat. No. 9,907,480, entitled "Catheter Spine Assembly with Closely-Spaced Bipole Microelectrodes," issued Mar. 6, 2018, the disclosure of which is incorporated by reference herein in its entirety; U.S. Pat. No. 10,130,422, entitled "Catheter with Soft Distal Tip for Mapping and Ablating Tubular Region," issued Nov. 20, 2018, the disclosure of which is incorporated by reference herein in its entirety; and U.S. Pub. No. 2018/0056038, entitled "Catheter with Bipole Electrode Spacer and Related Methods," published Mar. 1, 2018, the disclosure of which is incorporated by reference herein in its entirety.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3® system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued Nov. 1, 2016, the disclosure of which is incorporated by reference herein in its entirety; and various other references that are cited herein.

Cardiac mapping techniques have also been applied to detect electrically inactive tissue (e.g., scar tissue). This disclosure provides improved techniques for assessing scar tissue at different tissue depths
While various catheter systems and methods have been described, none are believed to provide the features described herein.

Summary of the Disclosure

This disclosure is directed to improved techniques for identifying scar tissue severity at various depths of cardiac tissue. Electrical activity is sensed from a plurality of electrodes of a multi-electrode catheter within a heart of a patient. For each of a plurality of surface locations of cardiac tissue in the heart, a processor identifies first electrical signals originating at a first depth within the cardiac tissue, identifies second electrical signals originating at a second depth within the cardiac tissue, estimates a first near-field signal originating at the first depth for each of the first electrical signals, detects scar borders at the first depth based on the first near-field signals originating at the first depth, estimates a second near-field signal originating at the second depth for each of the second electrical signals, and detects scar borders at the second depth based on the second near-field signals originating at the second depth. Based on the scar borders detected at the first and second depths, the processor visually displays first and second electro-anatomical maps of the heart depicting scar tissue at the first and second depths, respectively.

In some examples, the processor further estimates a first far-field signal originating at the first depth for each of the first electrical signals, and a second far-field signal originating at the second depth for each of the second electrical signals. In some such examples, the first and second far-field signals are estimated using weightings that decrease as a distance from a selected location increases.

In some examples, the multi-electrode catheter is comprised of unipolar electrodes. In some such examples, the unipolar electrodes are ring electrodes.

Examples herein improve existing computer-based electroanatomical mapping systems. In particular, the disclosed techniques for identifying electrical signals at different depths within cardiac tissue and estimating near-field signals and scar borders originating at the different depths improve the functioning of the computer itself enabling more accurate and insightful maps than could be generated using conventional systems or manual human analysis. Such improvements are rooted in technology and address problems unique to electrophysiological signal processing, including cancellation of far-field interference and enhancement of local activation detection.

Brief Description Of The Drawings

A more detailed understanding may be had from the following description, given by way of example in conjunction with the accompanying drawings, wherein like reference numerals in the figures indicate like elements, and wherein:

FIG. 1 shows an example catheter-based electrophysiology mapping and ablation system according to one or more embodiments;
FIG. 2 is a block diagram of an example system for remotely monitoring and communicating patient biometrics according to one or more embodiments;
FIG. 3 is a system diagram of an example of a computing environment in communication with a network according to one or more embodiments;
FIG. 4 shows an example of a multi-electrode catheter design for implementing techniques disclosed herein according to one or more embodiments;
FIG. 5 shows a further example of a multi-electrode catheter design for implementing techniques disclosed herein according to one or more embodiments;
FIG. 5A shows a further example of a multi-electrode catheter design for implementing techniques disclosed herein wherein the unipolar electrodes on the catheter of FIG. 5 are replaced with bi-polar split electrodes;
FIG. 6A shows a further example of a multi-electrode catheter design for implementing techniques disclosed herein according to one or more embodiments;

FIG. 6B shows a further example of a multi-electrode catheter design for implementing techniques disclosed herein according to one or more embodiments;

FIG. 7 depicts a portion of cardiac tissue to which the disclosed techniques are applied, in accordance with one or more embodiments;

FIG. 8 illustrates a technique for separately identifying the electrical activity at different cardiac tissue layers using a multi-electrode catheter according to one or more embodiments;

FIG. 9 is a flow chart illustrating a method to generate maps of cardiac scar severity at different tissue depths, according to an example of the present disclosure; and

Fig. 10 is a diagram that illustrates a method to estimate far-field from signals acquired using a multi-electrode catheter, according to an example of the present disclosure.

## Detailed Description

This disclosure is directed to improved techniques for identifying scar tissue severity at various depths of cardiac tissue. In the disclosed techniques, electrical activity at different depths of cardiac tissue is determined and analyzed in order to separately measure and detect scar tissue severity at each of the various tissue depths.

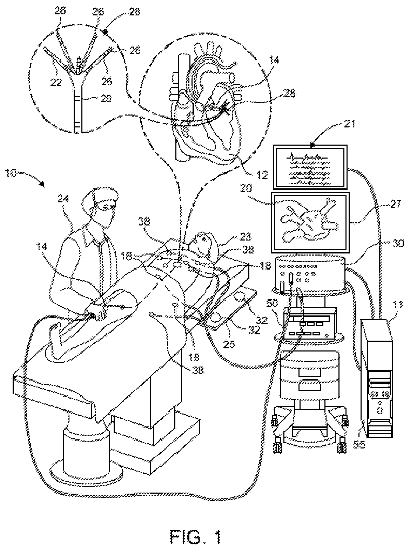

Reference is made to FIG. 1 showing an example system (e.g., medical device equipment and/or catheter-based electrophysiology mapping and ablation), shown as system 10, in which one or more features of the subject matter herein can be implemented according to one or more embodiments. The system 10, as illustrated, includes a recorder 11, a heart 12, a catheter 14, a model or anatomical map 20, an electrogram 21, a spline 22, a patient 23, a physician 24 (or a medical professional, healthcare provider, or clinician), a location pad 25, an electrode 26, a display device 27, a distal tip 28, a sensor 29, a coil 32, a patient interface unit (PIU) 30, an electrode skin patches 38, an ablation energy generator 50, and a workstation 55. Note further each element and/or item of the system 10 is representative of one or more of that element and/or that item. The example of the system 10 shown in FIG. 1 can be modified to implement the embodiments disclosed herein. The disclosed embodiments can similarly be applied using other system components and settings. Additionally, the system 10 can include additional components, such as elements for sensing electrical activity, wired or wireless connectors, processing and display devices, or the like.

The system 10 includes multiple catheters 14, which are percutaneously inserted by the physician 24 through the patient's vascular system into a chamber or vascular structure of the heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters 14 may include catheters dedicated for sensing Intracardiac Electrogram (IEGM) signals, catheters dedicated for ablating and/or catheters dedicated for both sensing and ablating. The example catheter 14 that is configured for sensing IEGM is illustrated herein. The physician 24 brings the distal tip 28 of the catheter 14 into contact with the heart wall for sensing a target site in the heart 12. For ablation, the physician 24 would similarly bring a distal end of an ablation catheter to a target site for ablating.

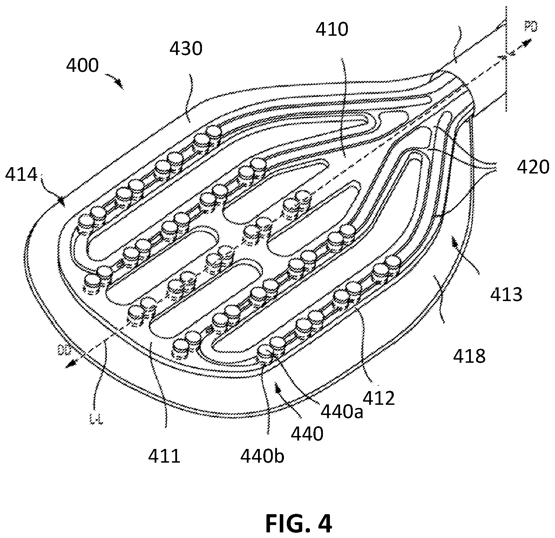

In FIG. 1, catheter 14 is depicted as including multiple electrodes 26 optionally distributed over a plurality of splines 22 at the distal tip 28 and configured to sense the IEGM signals. In some examples, catheter 14 is a multi-electrode configured to sense cardiac signals. Exemplary multi-electrode catheters for implementing the mapping techniques disclosed herein are shown in FIGs. 4-6 and discussed in further detail below. The catheter 14 may additionally include the sensor 29 embedded in or near the distal tip 28 for tracking position and orientation of the distal tip 28. Optionally and preferably, position sensor 29 is a magnetic based position sensor including three magnetic coils for sensing three-dimensional (3D) position and orientation. The catheter 14 may be a Pulsed-Field Ablation (PFA) catheter.

The sensor 29 (e.g., a position or a magnetic based position sensor) may be operated together with the location pad 25 including a plurality of magnetic coils 32 configured to generate magnetic fields in a predefined working volume. Real time position of the distal tip 28 of the catheter 14 may be tracked based on magnetic fields generated with the location pad 25 and sensed by the sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38 positioned for skin contact on the patient 23 to establish location reference for the location pad 25 as well as impedance-based tracking of the electrodes 26. For impedance-based tracking, electrical current is directed toward the electrodes 26 and sensed at the patches 38 (e.g., electrode skin patches) so that the location of each electrode can be triangulated via the patches 38. Details of the impedance-based location tracking technology are described in US Patent Nos. 7,536,218; 7,756,576; 7,848,787; 7,869,865; and 8,456,182, which are incorporated herein by reference.

The recorder 11 displays the electrograms 21 captured with the electrodes 18 (e.g., body surface electrocardiogram (ECG) electrodes) and intracardiac electrograms (IEGM) captured with the electrodes 26 of the catheter 14. The recorder 11 may include pacing capability for pacing the heart rhythm and/or may be electrically connected to a standalone pacer.

The system 10 may include the ablation energy generator 50 that is adapted to conduct ablative energy to the one or more of electrodes 26 at the distal tip 28 of the catheter 14 configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The PIU 30 is an interface configured to establish electrical communication between catheters, electrophysiological equipment, power supply and the workstation 55 for controlling operation of the system 10. Electrophysiological equipment of the system 10 may include for example, multiple catheters 14, the location pad 25, the body surface ECG electrodes 18, the electrode patches 38, the ablation energy generator 50, and the recorder 11. Optionally and preferably, the PIU 30 additionally includes processing capability for implementing real-time computations of location of the catheters and for performing ECG calculations.

The workstation 55 includes memory, processor unit with memory or storage with appropriate operating software loaded therein, and user interface capability. The workstation 55 may provide multiple functions, optionally including (1) modeling the endocardial anatomy in three-dimensions (3D) and rendering the model or anatomical map 20 for display on the display device 27, (2) displaying on the display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on the display device 27 sites of interest such as places where ablation energy has been applied. One commercial product embodying elements of the system 10 is available as the CARTOTM 3 System, available from Biosense Webster, Inc., 31A Technology Drive, Irvine, CA 92618.

For instance, the system 10 can be part of a surgical system (e.g., CARTO® system sold by Biosense Webster) that is configured to obtain biometric data (e.g., anatomical and electrical measurements of a patient's organ, such as the heart 12 and as described herein) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 12. Such locating may be done via an electrophysiological investigation during which electrical potentials are detected spatially resolved with a mapping catheter (e.g., the catheter 14) introduced into the chamber of the heart 12. This electrophysiological investigation, the so-called electro-anatomical mapping, thus provides 3D mapping data which can be displayed on the display device 27. In many cases, the mapping function and a treatment function (e.g., ablation) are provided by a single catheter or group of catheters such that the mapping catheter also operates as a treatment (e.g., ablation) catheter at the same time.

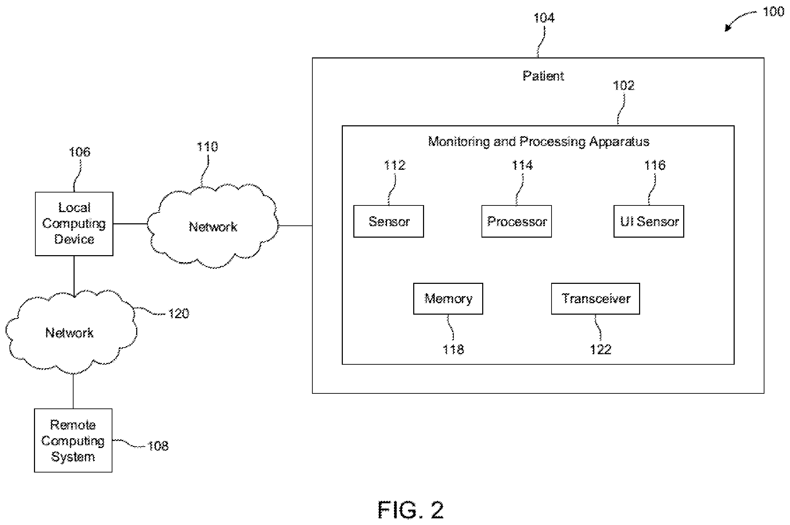

FIG. 2 is a block diagram of an example system 100 for remotely monitoring and communicating patient biometrics (i.e., patient data). In the example illustrated in FIG. 2, the system 100 includes a patient biometric monitoring and processing apparatus 102 associated with a patient 104, a local computing device 106, a remote computing system 108, a first network 110, a patient biometric sensor 112, a processor 114, a user input (UI) sensor 116, a memory 118, a second network 120, and a transmitter-receiver (i.e., transceiver) 122.

According to an embodiment, the patient biometric monitoring and processing apparatus 102 may be an apparatus that is internal to the patient's body (e.g., subcutaneously implantable), such as the catheter 14 of FIG. 1. The patient biometric monitoring and processing apparatus 102 may be inserted into a patient via any applicable manner including orally injecting, surgical insertion via a vein or artery, an endoscopic procedure, or a laparoscopic procedure.

According to an embodiment, the patient biometric monitoring and processing apparatus 102 may be an apparatus that is external to the patient, such as the electrode patches 38 of FIG. 1. For example, as described in more detail below, the patient biometric monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, the patient biometric monitoring and processing apparatus 102 may include both components that are internal to the patient and components that are external to the patient.

The single patient biometric monitoring and processing apparatus 102 is shown in FIG. 2. Example systems may, however, may include a plurality of patient biometric monitoring and processing apparatuses. A patient biometric monitoring and processing apparatus may be in communication with one or more other patient biometric monitoring and processing apparatuses. Additionally or alternatively, a patient biometric monitoring and processing apparatus may be in communication with the network 110.

One or more patient biometric monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other patient biometric monitoring and processing apparatuses 102. The additional information may be, for example, diagnosis information and/or additional information obtained from an additional device such as a wearable device. Each of the patient biometric monitoring and processing apparatus 102 may process data, including its own acquired patient biometrics as well as data received from one or more other patient biometric monitoring and processing apparatuses 102.

Biometric data (e.g., patient biometrics, patient data, or patient biometric data) can include one or more of local activation times (LATs), electrical activity, topology, bipolar mapping, reference activity, ventricle activity, dominant frequency, impedance, or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, intracardiac electrocardiogram (IC ECG) data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treat any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

In FIG. 2, the network 110 is an example of a short-range network (e.g., local area network (LAN), or personal area network (PAN)). Information may be sent, via the network 110, between the patient biometric monitoring and processing apparatus 102 and the local computing device 106 using any one of various short-range wireless communication protocols, such as Bluetooth, Wi-Fi, Zigbee, Z-Wave, near field communications (NFC), ultraband, Zigbee, or infrared (IR).

The network 120 may be a wired network, a wireless network or include one or more wired and wireless networks. For example, the network 120 may be a long-range network (e.g., wide area network (WAN), the internet, or a cellular network,). Information may be sent, via the network 120 using any one of various long-range wireless communication protocols (e.g., TCP/IP, HTTP, 3G, 4G/LTE, or 5G/New Radio).

The patient biometric monitoring and processing apparatus 102 may include the patient biometric sensor 112, the processor 114, the UI sensor 116, the memory 118, and the transceiver 122. The patient biometric monitoring and processing apparatus 102 may continually or periodically monitor, store, process and communicate, via the network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

The patient biometric sensor 112 may include, for example, one or more sensors configured to sense a type of biometric patient biometrics. For example, the patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer or a microphone.

As described in more detail below, the patient biometric monitoring and processing apparatus 102 may be an ECG monitor for monitoring ECG signals of a heart (e.g., the heart 12). The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closed-loop system in which the blood glucose data is sent to an insulin pump for calculated delivery of insulin without user intervention.

The transceiver 122 may include a separate transmitter and receiver. Alternatively, the transceiver 122 may include a transmitter and receiver integrated into a single device.

The processor 114 may be configured to store patient data, such as patient biometric data in the memory 118 acquired by the patient biometric sensor 112, and communicate the patient data, across the network 110, via a transmitter of the transceiver 122. Data from one or more other patient biometric monitoring and processing apparatus 102 may also be received by a receiver of the transceiver 122, as described in more detail below.

According to an embodiment, the patient biometric monitoring and processing apparatus 102 includes a UI sensor 116 which may be, for example, a piezoelectric sensor or a capacitive sensor configured to receive a user input, such as tapping or touching. For example, the UI sensor 116 may be controlled to implement a capacitive coupling in response to tapping or touching a surface of the patient biometric monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from the patient biometric monitoring and processing apparatus 102 when a gesture is detected.

The local computing device 106 of the system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via the network 120. Alternatively, the local computing device 106 may be a stationary or standalone device, such as a stationary base station including, for example, modem and/or router capability, a desktop or laptop computer using an executable program to communicate information between the patient biometric monitoring and processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, the remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

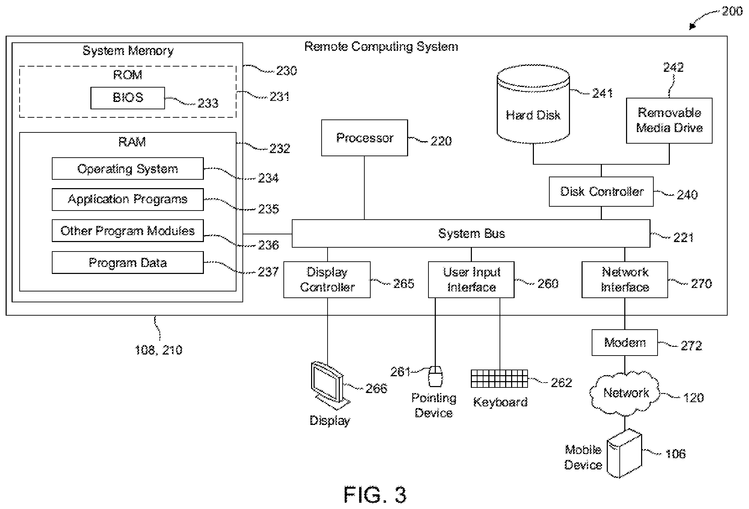

FIG. 3 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 3, computing environment 200 includes remote computing system 108 (hereinafter computer system), which is one example of a computing system upon which embodiments described herein may be implemented.

The remote computing system 108 may, via processors 220, which may include one or more processors, perform various functions. The functions may include analyzing monitored patient biometrics and the associated information and, according to physician-determined or algorithm-driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 3, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or all of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multi-processing arrangement to execute one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGS. 2 and 3, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 210 and other computers (e.g., local computing device 106).

A catheter ablation based treatment may include mapping the electrical properties of heart tissue, especially the endocardium and the heart volume, and selectively ablating cardiac tissue by application of energy. Cardiac mapping, for example creating a map of electrical potentials (a voltage map) of the wave propagation along the heart tissue or a map of arrival times (a local time activation (LAT) map) to various tissue located points, may be used for detecting local heart tissue dysfunction. Ablations, such as those based on cardiac mapping, can cease or modify the propagation of unwanted electrical signals from one portion of the heart to another.

The ablation process damages the unwanted electrical pathways by formation of non-conducting lesions. Cardiac ablation may rely on the use of three dimensional (3D) mapping systems, for example CARTO®3 3D mapping system, produced by Biosense Webster, Inc. (Diamond Bar, Calif). The 3D maps can provide multiple pieces of information including visualization of tags (e.g. on a display or monitor) representing the location of electrodes of a multi-electrode catheter for an ablation session.

While the techniques described herein are not limited to any particular multi-electrode electrode catheter arrangement, the techniques may be understood in connection with the multi-electrode catheter designs 400 and 500 discussed below.

FIG. 4 shows an example showing an end effector 400 for a catheter. The end effector 400 can include a flexible circuit layer 410 extending along a longitudinal axis L- L from a proximal portion 413 to a distal portion 414, a framework 420 coupled to the flexible circuit layer 410 and extending generally parallel to the flexible circuit layer 410 along the longitudinal axis L-L from the proximal 413 to the distal portion 414, and a flexible polymer layer 430 encapsulating both the framework 420 and the flexible circuit layer 410. The flexible circuit layer 410 can include a first surface 411 and a second surface 412 opposite the first surface 411. The flexible circuit layer 410 defines a planar configuration 418 extending through the longitudinal axis L-L with the framework 420 disposed inside the flexible circuit layer 410.

In some examples, the end effector 400 can include one or more first electrodes 440a affixed to the first surface 411 of the flexible circuit layer 410, and one or more second electrodes 440b affixed to the second surface 412 of the flexible circuit layer 410. The first electrodes 440a can be axially aligned with the second electrodes 440b to define pairs of opposite facing electrodes 440. In some examples, pairs of opposite facing electrodes 440 can be aligned generally parallel to the longitudinal axis L- L. In other examples, pairs of opposite facing electrodes 440 can be aligned generally transverse to the longitudinal axis L-L. Further details of end effector 400 are set forth in U.S. Patent Application No. 18/505,956 entitled "Catheter With Flexible Polymer As Outer Support Structure," filed November 9, 2023, incorporated herein in its entirety by reference.

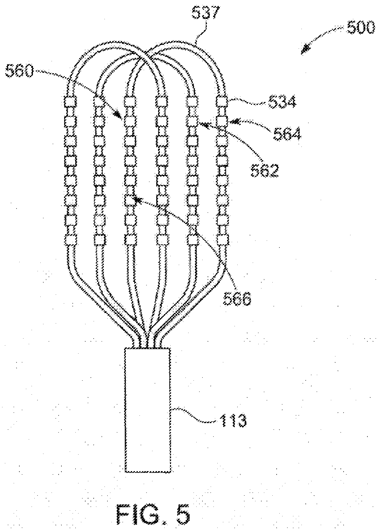

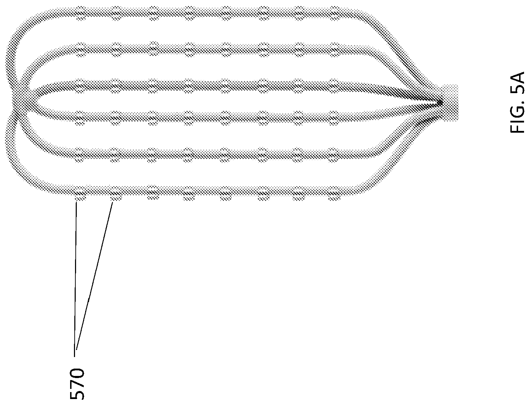

FIG. 5 shows a further example of a multi-electrode catheter design 500 for implementing techniques disclosed herein according to one or more embodiments. The catheter 500 can be a catheter having a plurality of electrodes 534, such as at least twenty (20) electrodes. As shown, the plurality of electrodes can include exactly forty eight (48) electrodes located across or dispersed on multiple spines 537. Using such a number of electrodes 534 spread across a wide area by the spines 537 allows the capturing of large amount of electrical activity over a large area at once. According to one or more embodiments, the multiple spines 537 move through a sheath in a collapsed state and may be expanded once within the patient 23 of FIG. 1 . Electrical activity at any focal point in the heart 12 may be typically measured by advancing the catheter 500, contacting the heart tissue with the catheter 500, and acquiring data related to electrical activity at that point.

As shown, catheter 500 is formed from an array of unipolar electrodes. In one embodiment, the unipolar electrodes are ring electrodes. The electrodes are considered to be unipolar, because the voltage may be measured at each electrode relative to a common electrode positioned, e.g., on the surface of the patient's body, or relative to a reference electrode on the catheter. In alternate designs, the unipolar electrodes on catheter 500 may be replaced with bi-polar electrodes 570 as illustrated in FIG. 5A In yet other alternate designs, the unipolar electrodes on catheter 500 may be selectively paired to form bi-polar electrodes in real-time, as described in U.S. Patent Application No. 17/341,315, entitled "Bipolar Electrode Pair Selection," incorporated herein by reference.

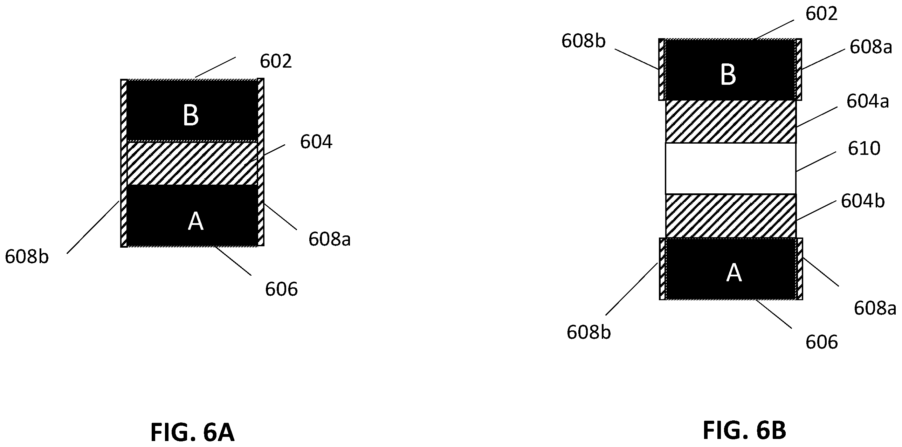

As noted above, catheter 400 includes two co-planar arrays of electrodes, one array being on each side (or face) of the catheter. In one embodiment, the electrodes on each face of catheter 400 are arranged in pairs and referred to as bi-polar electrodes, because the voltage is measured between each bi-polar pair. In some arrangements, a dielectric layer 604 separates the faces of catheter 400, as shown in FIGs. 6A and 6B. In this arrangement, the bi-polar electrode pairs on each face of catheter 400 are either replaced with, or used to model, electrode pairs where the electrodes in each pair are on either side of the dielectric layer separating the faces of the catheter. An example of one such electrode pair (comprised of electrodes 602, 606) where the electrodes in the pair are on either side of the dielectric layer 604 separating the faces of the catheter is shown in FIG. 6A. A further example of one such electrode pair (comprised of electrodes 602, 606) is shown in FIG. 6B. In the example of FIG. 6B, a dielectric layer 604a, 604b is positioned adjacent to each of electrodes 602, 606, and other components 610 of the catheter (e.g., one or more catheter substrates, electrical traces, polymer or other materials) are positioned between dielectric layers 604a, 604b. In the electrode arrangements shown in FIGs. 6A and 6B, dielectric material 608a, 608b are optionally disposed along the sidewalls of electrodes 602, 606.

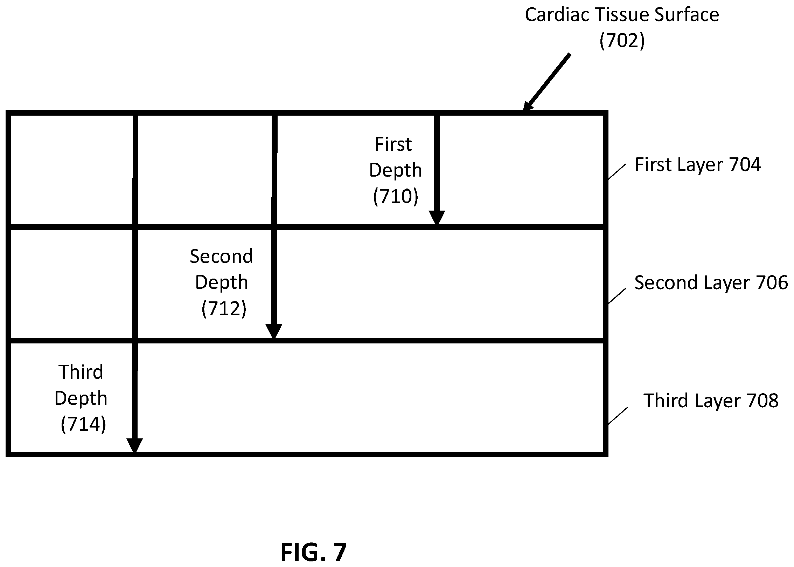

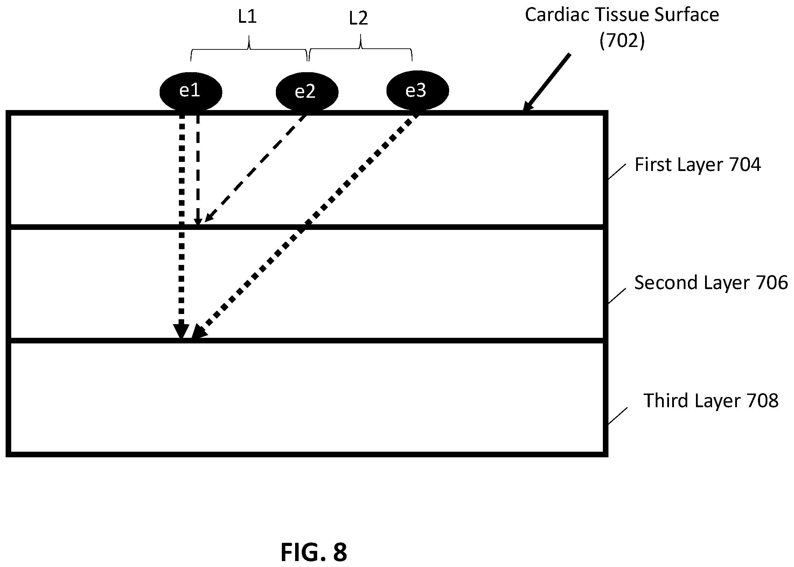

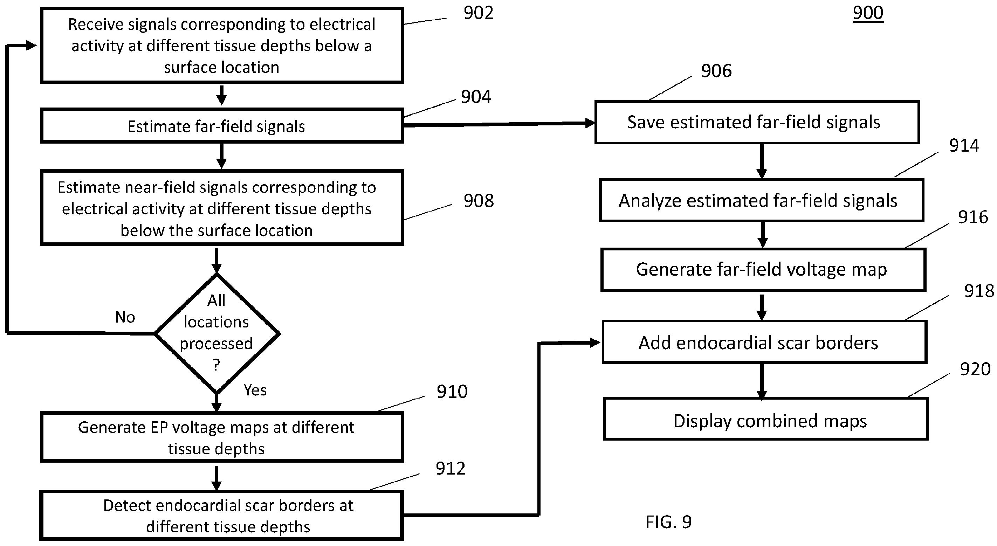

In some examples, the signal analysis described in connection with FIGs. 7 and 8 below is performed and the results are applied to method described in connection with FIG. 9 in order to generate maps of cardiac scar severity at different tissue depths. Referring now to FIG. 7, a portion of cardiac tissue is depicted in which the cardiac tissue surface 702 (which may come into direct contact with an electrode or be proximate thereto during a medical procedure) is at the top. Below surface 702 are three tissue layers 704, 706, 708, each of which is positioned at a different depth (710, 712, 714) from the tissue surface 702.

Referring now to FIG. 8, a technique is shown for separately identifying electrical activity at different tissue layers 704, 706, 708 using a multi-electrode catheter with electrodes (labelled e1, e2 and e3). In the diagram, electrodes e1 and e2 are separated by a distance (L1), and electrodes e2 and e3 are separated by a distance (L2). For purposes of simplicity, the calculations set forth below assume that L1=L2; however, it will be understood that the electrodes need not be equally spaced. In the calculations below, the far-field voltages at electrodes e1, e2 and e3 correspond to $V1_{ff}$, $V2_{ff}$ and $V3_{ff}$, respectively. For simplicity, the far-field effect from Layer 1 (Vff layer 1) is assumed to be $V1_{ff}$ - $V2_{ff}$; and the far-field effect from Layer 1 and Layer 2 combined is assumed to be $V1_{ff}$ - $V3_{ff}$. In more sophisticated implementations, the far-field voltages collected at e1, e2 and e3 may be processed, weighted and/or filtered to remove or adjust certain signal components prior to performing calculations using this technique. In order to determine the far-field effect from Layer 2 only (Vff layer 2), the following equation is used:

$$Far-field\ Effect\ of\ Layer\ 2\ Alone = \left(V1_{ff} - V3_{ff}\right) - \beta * \left(V1_{ff} - V2_{ff}\right),\ \beta \approx 2$$

Similarly, in order to isolate the far-field effect from Layer 3 only ($V_{ff\ layer\ 3}$), the following equation is used:

$$Far-field\ Effect\ of\ Layer\ 3\ Alone = \left(V3_{ff} - V4_{ff}\right) - \lambda * \left(V1_{ff} - V3_{ff}\right),\ \lambda \approx 3$$

FIG. 9 is a flow chart illustrating a method 900 to generate EP maps of cardiac scar severity at different tissue depths, according to an example of the present disclosure. The algorithm carries out a process for assessing scar severity at different depths from a first tissue surface location by initially (in step 902) receiving signals $V_{fflayer1}$, $V_{fflayer2}$ and $V_{fflayer3}$ (corresponding to far-field effects from different depths from the first tissue surface location) as discussed above in connection with FIG. 8. The processor may be processor in workstation 55 of an offline computer. At far-field signal estimation step 904, the processor estimates far-field signals from the signals received at step 902 by, for example, applying the techniques described in U.S. Patent Application Publication 2023/0181087 to each of the different layers of cardiac tissue. The processor saves the far-field signals for use with the disclosed algorithm at a far-field signal-saving step 906. In near-field signals estimation step 908, the processor estimates the near-field signal activity at each of the different tissue depths below the surface location by subtracting the far-field signal components associated with each layer ($V_{ff\ layer\ n}$ calculated using the formulas set forth above). In some examples, the near-field activity is initially estimated for the layer immediately adjacent to the electrode, which is a shallow layer of, e.g., the endocardium. In some examples, a shallow endocardium scar is identified from the near-field at the shallow layer immediately adjacent to the electrode, and the voltage values for the layers at deeper depths are generated by subtracting the far-field signal components associated with each layer ($V_{ff\ layer\ n}$ calculated using the formulas set forth above) from the near-field signal detected for the shallow layer. If the calculation shows very low far-field effects at the deeper layers, it means the scar tissue extends from the shallow layer to within those deeper layers as well. Steps 902-908 are repeated for a plurality of locations. In step 910, EP voltage maps are generated at each of the different layers of cardiac tissue using near-field signals resulting from step 906. For each EP map associated with a different layer of cardiac tissue, the processor detects the endocardial scar regions borders in step 912. In parallel, at an analysis step 914, for each of the different tissue depths below the surface location, the processor analyzes the far field signals saved in step 906 to estimate scar locations and depths. The processor relies on the far-field signals being at certain wall tissue locations slightly different (e.g., smaller) than the total signal as a function of location and depth. The method defines scar severity as the normalized ratio of scar width to total tissue width. It estimates the ratio based on (i) a mathematical model that analyzes the far-field signal and/or spatial derivatives and (ii) correspondences obtained empirically between the far-field EP signals and anatomical dissections or MRI imaging of actual scars. Using the analysis done in step 914, for each of the different tissue depths below the surface location, the processor generates a far-field EP map in step 916. Each of these EP maps may be a voltage map or a voltage gradient map, to name two examples. In step 920, for each of the different tissue depths below the surface location, the processor adds (e.g., overlays) borders detected in step 912 on the corresponding far-field EP map generated in step 916. The resulting maps (one for each tissue depth) are referred to as "scar severity maps." Finally, in step 920, the processor displays the combined maps (i.e., the scar severity map) to a user.

As mentioned above, one method to estimate and remove unipolar far-field signals is described in U.S. Patent Application Publication 2023/0181087, which is assigned to the assignee of the present disclosure and incorporated herein in its entirety by reference. The algorithm disclosed in U.S. Patent Application Publication 2023/0181087 is inspired by an assumption that intracardiac unipolar far-field cardiac EP signals are similar but not the same on all multi-electrode EP mapping catheter electrodes. In the technique described therein, a mapping engine (e.g., a processor running the algorithm) weights catheter electrodes in opposite proportion to distances to identify a common signal (e.g., a far field signal) component across the electrodes. The mapping engine uses this common signal component to reduce or cancel far-field interference precisely. Reducing or canceling the far-field interference by the mapping engine produces cleaner near-field signals for improved localized (e.g., near-field) cardiac mapping, as described in the above-mentioned patent application. The present disclosure extends the techniques described in the above-mentioned patent application to analysis of signals at different depths in the cardiac tissue, by separately performing the far-field signal reduction or cancellation techniques at multiple different depths of cardiac tissue based on electrical activity detected at different depths from a tissue surface location (e.g., Vlayer1, Vlayer2 and Vlayer3 as discussed above in connection with FIG. 8.)

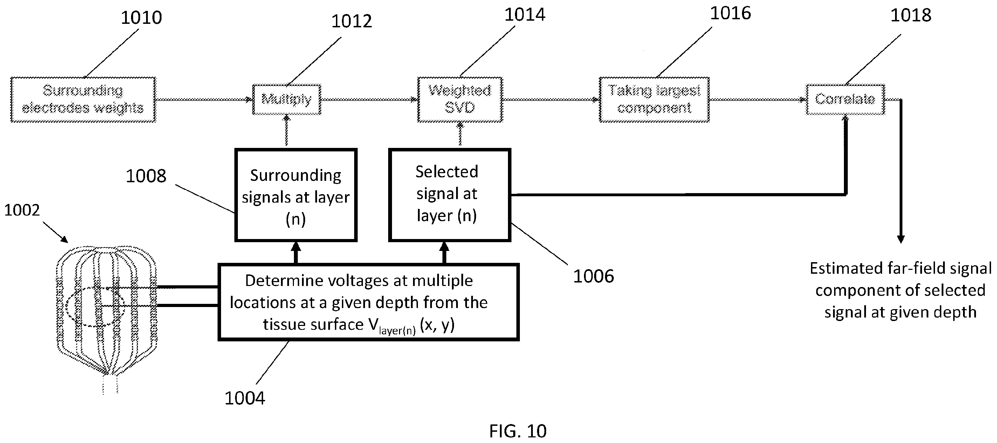

Fig. 10 is a diagram that illustrates a method to estimate far-field from signals acquired using a multi-electrode catheter, such as the OPTRELL™ catheter 1002, according to an example of the present disclosure. In step 1004, the techniques described in connection with FIG. 8 above are applied to determine electrical signals (Vlayer(n) (x, y)) at a given depth (layern) in the cardiac tissue. In step 1006, one of the electrical signals (the "selected signal") calculated for a selected spatial location (x, y) (the "selected location") in the given depth (layern) is selected. In step 1008, electrical signals (the "surrounding signals") for locations surrounding (the "surrounding locations") the selected spatial location in the given depth (layern) are selected. In the disclosed example, where the depth of the given depth layer is measured along the z-axis running perpendicular to the tissue surface, the selected location and the surrounding locations corresponding to the signals selected in steps 1006 and 1008 are all in the given depth (layern) and/or at the same level on the z-axis. In step 1010, a processor weights each surrounding signal in opposite proportion to a distance between the surrounding location associated with such surrounding signal and the selected location. In step 1012, the processor multiplies the surrounding signals by the respective weights. A matrix of the weighted far-field signals contribution for each surrounding location is generated (step 1014) and the largest signal is estimated (step 1016). The processor correlates (step 1018) the largest signal with the selected signal to provide the far field estimation for the selected signal at the given depth.

The techniques disclosed herein are particularly applicable for characterizing scar tissue at different depths. Scar tissue typically corresponds to areas of tissue where conductivity is low. In one example, differences (gradients) between far field signals are calculated for different electrodes. Differences may include differences in signal amplitude and signal shape (particularly if the electrodes are far from each other). Using any of the previous applicable techniques, far field signals of nearby electrodes are subtracted to provide information for characterizing tissue (and signals) at shallow depths, while far field signals of electrodes further away are subtracted to provide information for characterizing tissue (and signals) at deeper depths. These signal processing techniques can then be used to generate 3D maps of, e.g., wave amplitudes at different depths of the tissue, and more specifically characterize scar tissue by tissue depth, e.g., identify tissue location and depth where there is low energy. The techniques can be applied using unipolar or bipolar electrodes.

The above-described concepts may involve generating electro-anatomical maps that depict electrical activity within a three-dimensional volume of the cardiac tissue. As noted, these maps may be organized in "layers," each corresponding to a different depth beneath the surface of the cardiac tissue. For example, the system may display layer 1 representing the electrical activity closest to the surface of the tissue, layer 2 representing a deeper region, and layer 3 showing the deepest electrical activity. By visualizing electrical activity across these different depths, the system may allow for a more comprehensive understanding of the tissue's electrical properties throughout its thickness, similar to peeling back layers of an onion. This multi-layered view is particularly beneficial for identifying electrical abnormalities within the deeper layers of the myocardium, such as identifying areas of scarring or zones of arrhythmogenic tissue that may not be visible from surface-level maps. Such insights can significantly enhance the physician's ability to locate arrhythmia sources and optimize ablation therapy.

To facilitate the selection and visualization of these map layers, the system may offer several user interface (UI) and user experience (UX) design options. One method could involve a menu that allows the operator to select specific layers, such as "Layer 1," "Layer 2," or "Layer 3," to view electrical activity at varying depths. Alternatively, a more dynamic approach could involve using a scrolling mechanism, similar to zooming in or out with a mouse or trackpad. As the operator scrolls, the system could progressively reveal deeper layers of the map, effectively peeling back the layers of tissue to show electrical activity at successively greater depths. Alternatively or additionally, layers of depths of the map volume may be revealed dynamically about a region as the operator of the mapping system navigates about the map (e.g., a region beneath the cursor may expose the inner layers of the map).

When scar tissue regions are identified, these depth-based layers could be limited to the regions of the scar, allowing for a detailed three-dimensional representation of the scar tissue within the myocardium. This layered, depth-based view can provide a more precise understanding of scar morphology, helping physicians better plan and execute ablation procedures in arrhythmia treatment.

Although features and elements are described above in particular combinations, one of ordinary skills in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

Aspects of the invention:

1. A method, comprising:

(a) receiving electrical activity from a plurality of electrodes of a multi-electrode catheter within a heart of a patient;
(b) for a first surface location of cardiac tissue in the heart:

(i) identifying first electrical signals originating at a first depth from the first surface location;
(ii) identifying second electrical signals originating at a second depth within the cardiac tissue;

(c) repeating step (b) for a plurality of further surface locations of cardiac tissue;
(d) for the first surface location and each of the plurality of further surface locations:

(i) estimating a first near-field signal originating at the first depth for each of the first electrical signals;
(ii) detecting scar borders at the first depth based on the first near-field signals originating at the first depth;
(iii) estimating a second near-field signal originating at the second depth for each of the second electrical signals; and
(iv) detecting scar borders at the second depth based on the second near-field signals originating at the second depth;

(e) based on the scar borders detected at the first depth, visually displaying a first electro-anatomical map of the heart depicting scar tissue at the first depth; and
(f) based on the scar borders detected at the second depth, visually displaying a second electro-anatomical map of the heart depicting scar tissue at the second depth.

2. The method according to aspect 1, further comprising:

estimating a first far-field signal originating at the first depth for each of the first electrical signals; and
estimating a second far-field signal originating at the second depth for each of the second electrical signals.

3. The method of aspect 2, wherein the first and second far-field signals are estimated using weightings that decrease as a distance from a selected location increases.

4. The method of aspect 1, where the multi-electrode catheter is comprised of unipolar electrodes.

5. The method of aspect 1, where the multi-electrode catheter is comprised of unipolar electrodes that are ring electrodes.

6. The method of aspect 1, wherein at least one of the plurality of electrodes is in contact with the cardiac tissue at the first location.

7. The method of aspect 1, wherein the multi-electrode catheter is comprised of first, second and third electrodes in contact with a cardiac tissue surface, wherein the first electrode senses a first voltage, the second electrode senses a second voltage and the third electrode senses a third voltage, wherein the first electrode and the second electrode are separate by a first length, and the second and third electrodes are separated by a second length.

8. The method of aspect 7, wherein the first length is equal to the second length and wherein the first electrical signals originating at the first depth from the first surface location are determined based on a difference between the first voltage and the second voltage; and the second electrical signals originating at the second depth are determined based on a difference between the second voltage and the third voltage.

9. The method of aspect 6, wherein the second electrical signals originating at the second depth are determined based on the following equation:

$$Far-field\ Effect\ of\ Layer\ 2\ Alone = (V1_{ff} - V3_{ff}) - \beta * (V1_{ff} - V2_{ff}),\ \ \beta \approx 2$$

wherein $V1_{ff}$, $V2_{ff}$ and $V3_{ff}$ respectively. correspond to far field signals received at the first electrode, the second electrode and the third electrode, respectively, and $\beta$ is a coeeficient.

**Claims**

1. A system comprising:

one or more processors in communication with a plurality of electrodes of a multi-electrode catheter;
a display; and
a memory in communication with the display and the one or more processors;
wherein the one or more processors are collectively configured to:

receive electrical activity from a plurality of electrodes of a multi-electrode catheter within a heart of a patient;
for each of a plurality of surface locations of cardiac tissue in the heart:

identify first electrical signals originating at a first depth within the cardiac tissue;
identify second electrical signals originating at a second depth within the cardiac tissue;
estimate a first near-field signal originating at the first depth for each of the first electrical signals;
detect scar borders at the first depth based on the first near-field signals originating at the first depth;
estimate a second near-field signal originating at the second depth for each of the second electrical signals; and
detect scar borders at the second depth based on the second near-field signals originating at the second depth;

based on the scar borders detected at the first depth, visually display a first electro-anatomical map of the heart depicting scar tissue at the first depth; and
based on the scar borders detected at the second depth, visually display a second electro-anatomical map of the heart depicting scar tissue at the second depth.

2. The system according to claim 1, wherein the one or more processors are further collectively configured to:

estimate a first far-field signal originating at the first depth for each of the first electrical signals; and
estimate a second far-field signal originating at the second depth for each of the second electrical signals.

3. The system of claim 2, wherein the first and second far-field signals are estimated using weightings that decrease as a distance from a selected location increases.

4. The system of claim 1, where the multi-electrode catheter is comprised of unipolar electrodes.

5. The system of claim 1, where the multi-electrode catheter is comprised of unipolar electrodes that are ring electrodes.

6. The system of claim 1, wherein at least one of the plurality of electrodes is in contact with the cardiac tissue at the first location.

7. The system of claim 1, wherein the multi-electrode catheter is comprised of first, second and third electrodes in contact with a cardiac tissue surface, wherein the first electrode senses a first voltage, the second electrode senses a second voltage and the third electrode senses a third voltage, wherein the first electrode and the second electrode are separate by a first length, and the second and third electrodes are separated by a second length.

8. The system of claim 7, wherein the first length is equal to the second length, the first electrical signals originating at the first depth from the first surface location are determined based on a difference between the first voltage and the second voltage; and the second electrical signals originating at the second depth are determined based on a difference between the second voltage and the third voltage.

9. The system of claim 7, wherein the second electrical signals originating at the second depth are determined based on the following equation:

$$Far-field\ Effect\ of\ Layer\ 2\ Alone = \left(V1_{ff} - V3_{ff}\right) - \beta * \left(V1_{ff} - V2_{ff}\right),\ \ \beta \approx 2$$

wherein $V1_{ff}$, $V2_{ff}$ and $V3_{ff}$, respectively. correspond to far field signals received at the first electrode, the second electrode and the third electrode, respectively, and $\beta$ is a coeeficient.

10. A computer-readable storage medium comprising program code for causing a computer to:

receive electrical activity from a plurality of electrodes of a multi-electrode catheter within a heart of a patient;
for each of a plurality of surface locations of cardiac tissue in the heart:

identify first electrical signals originating at a first depth within the cardiac tissue;
identify second electrical signals originating at a second depth within the cardiac tissue;
estimate a first near-field signal originating at the first depth for each of the first electrical signals;
detect scar borders at the first depth based on the first near-field signals originating at the first depth;
estimate a second near-field signal originating at the second depth for each of the second electrical signals; and
detect scar borders at the second depth based on the second near-field signals originating at the second depth;

based on the scar borders detected at the first depth, visually display a first electro-anatomical map of the heart depicting scar tissue at the first depth; and
based on the scar borders detected at the second depth, visually display a second electro-anatomical map of the heart depicting scar tissue at the second depth.

11. The computer-readable storage medium of claim 10, where the multi-electrode catheter is comprised of unipolar electrodes.

FIG. 1

100

104 Patient

102 Monitoring and Processing Apparatus

112 Sensor

114 Processor

116 UI Sensor

118 Memory

122 Transceiver

110 Network

106 Local Computing Device

120 Network

108 Remote Computing System

**FIG. 2**

FIG. 3

**FIG. 4**

EP 4 714 354 A1

FIG. 5

EP 4 714 354 A1

FIG. 5A

570

FIG. 6B

FIG. 6A

Cardiac Tissue Surface
(702)

First Depth
(710)

First Layer 704

Second
Depth
(712)

Second Layer 706

Third
Depth
(714)

Third Layer 708

EP 4 714 354 A1

FIG. 7

**FIG. 8**

EP 4 714 354 A1

EP 4 714 354 A1

```
┌─────────────────────────────────┐
│ Receive signals corresponding   │──── 902          900
│ to electrical activity at        │
│ different tissue depths below a  │
│ surface location                 │
└─────────────────────────────────┘                906
              │
              ▼                          ┌──────────────────────────────┐
┌─────────────────────────────────┐ 904  │ Save estimated far-field     │
│ Estimate far-field signals      │─────▶│ signals                      │
└─────────────────────────────────┘      └──────────────────────────────┘
              │                                        │           914
              ▼                                        ▼
┌─────────────────────────────────┐      ┌──────────────────────────────┐
│ Estimate near-field signals      │      │ Analyze estimated far-field   │
│ corresponding to electrical      │ 908  │ signals                       │
│ activity at different tissue     │      └──────────────────────────────┘
│ depths below the surface location│                  │           916
└─────────────────────────────────┘                  ▼
              │                          ┌──────────────────────────────┐
              ▼                          │ Generate far-field voltage map│
           ◇ All ◇                       └──────────────────────────────┘
     No  ◇ locations ◇                               │           918
    ◀────◇ processed ◇                               ▼
         ◇   ?   ◇                       ┌──────────────────────────────┐
              │ Yes                       │ Add endocardial scar borders  │
              ▼              910         └──────────────────────────────┘
┌─────────────────────────────────┐                  │           920
│ Generate EP voltage maps at      │                  ▼
│ different tissue depths          │      ┌──────────────────────────────┐
└─────────────────────────────────┘      │ Display combined maps         │
              │              912         └──────────────────────────────┘
              ▼
┌─────────────────────────────────┐
│ Detect endocardial scar borders  │
│ at different tissue depths       │
└─────────────────────────────────┘
```

FIG. 9

EP 4 714 354 A1

1010     1012     1014     1016     1018

| Surrounding electrodes weights | → | Multiply | → | Weighted SVD | → | Taking largest component | → | Correlate |

1008

Surrounding signals at layer (n)

Selected signal at layer (n)

1002

1006

Determine voltages at multiple locations at a given depth from the tissue surface $V_{layer(n)}(x, y)$

1004

Estimated far-field signal component of selected signal at given depth

FIG. 10

EUROPEAN SEARCH REPORT

Application Number

EP 25 20 3761

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 4 670 628 A1 (BIOSENSE WEBSTER ISRAEL LTD [IL]) 31 December 2025 (2025-12-31) <br> * abstract; figures 1,8 * <br> * paragraph [0107] - paragraph [0108] * <br> * paragraph [0112] - paragraph [0113] * <br> ----- | 1-8,10, 11 | INV. <br> A61B5/287 <br> A61B5/367 <br> A61B5/00 <br> A61B18/14 |
| X | US 2020/281494 A1 (ELIYAHU SHIRAN [IL] ET AL) 10 September 2020 (2020-09-10) <br> * abstract; figures 1-6 * <br> * paragraph [0039] - paragraph [0041] * <br> * paragraph [0058] * <br> * paragraph [0060] * <br> * paragraph [0064] - paragraph [0065] * <br> * paragraph [0094] * <br> ----- | 1-11 | |
| X | BEHESHTI MOHAMMADALI ET AL: "Determinants of atrial bipolar voltage: Inter electrode distance and wavefront angle", COMPUTERS IN BIOLOGY AND MEDICINE, NEW YORK, NY, US, vol. 102, 27 July 2018 (2018-07-27), pages 449-457, XP085515312, ISSN: 0010-4825, DOI: 10.1016/J.COMPBIOMED.2018.07.011 <br> * abstract; figures 3,6,7 * <br> * Section 3.4 * <br> ----- | 1-11 | |

TECHNICAL FIELDS SEARCHED (IPC)

A61B

The present search report has been drawn up for all claims

| Place of search | Date of completion of the search | Examiner |
|---|---|---|
| Berlin | 23 January 2026 | Sleightholme, G |

EPO FORM 1503 03.82 (P4C01)

## ANNEX TO THE EUROPEAN SEARCH REPORT
## ON EUROPEAN PATENT APPLICATION NO.

EP 25 20 3761

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2026

| Patent document cited in search report | | Publication date | Patent family member(s) | | Publication date |
|---|---|---|---|---|---|
| EP 4670628 | A1 | 31-12-2025 | CN | 121221124 A | 30-12-2025 |
| | | | EP | 4670628 A1 | 31-12-2025 |
| | | | IL | 321747 A | 01-01-2026 |
| | | | JP | 2026008941 A | 19-01-2026 |
| US 2020281494 | A1 | 10-09-2020 | CN | 111657913 A | 15-09-2020 |
| | | | EP | 3705037 A1 | 09-09-2020 |
| | | | IL | 273008 A | 30-09-2020 |
| | | | JP | 7536473 B2 | 20-08-2024 |
| | | | JP | 2020142079 A | 10-09-2020 |
| | | | US | 2020281494 A1 | 10-09-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63697933
- US 07279322
- US 75690324
- US 23879125
- US 50595623
- US 20130030426 A
- US 20170312022 A
- US 20180071017 A
- US 20180056038 A
- US 10130422 B
- US 8956353 B
- US 9801585 B
- US 5738096 A
- US 9907480 B
- US 9480416 B
- US 55391199 B
- US 5443489 A

- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B
- US 341315
- US 20230181087